# EUROPEAN PATENT SPECIFICATION

(11) **EP 1 832 288 B1**
(45) Date of publication and mention of the grant of the patent: **20.06.2012**
(21) Application number: 05820232.6
(22) Date of filing: 26.12.2005
(51) Int. Cl.: A61K 9/20, A61K 31/4725, A61K 9/16

(54) **STABLE GRANULAR PHARMACEUTICAL COMPOSITION OF SOLIFENACIN OR ITS SALT**
STABILE GRANULATFÖRMIGE PHARMAZEUTISCHE ZUSAMMENSETZUNG VON SOLIFENACIN ODER SEINEM SALZ
PRÉPARATION PHARMACEUTIQUE GRANULAIRE STABLE DE SOLIFÉNACINE OU DE SON SEL

(30) Priority: 27.12.2004 US 638388 P; 24.03.2005 JP 2005085968
(43) Date of publication of application: 12.09.2007
(73) Proprietor: Astellas Pharma Inc., Tokyo 103-8411 (JP)
(72) Inventor: UMEJIMA, Hiroyuki, Astellas Pharma Inc., Chuo-ku Tokyo 1038411 (JP); OHI, Hiroshi, Astellas Pharma Inc., Chuo-ku Tokyo 1038411 (JP); SAITO, Katsumi, Astellas Pharma Inc., Chuo-ku Tokyo 1038411 (JP); TAKETANI, Yuko, Astellas Pharma Inc., Chuo-ku Tokyo 1038411 (JP)
(74) Representative: HOFFMANN EITLE
(86) International application number: PCT/JP2005/023771
(87) International publication number: WO 2006/070735

(56) References cited:
- EP-A1- 1 552 825
- EP-A1- 1 728 791
- EP-A1- 1 832 288
- WO-A1-96/20194
- WO-A1-03/103659
- WO-A1-2005/092889
- JP-A- 2004 175 796
- JP-A- 2004 175 796
- US-A- 6 011 062

## Description

### Technical Field

The present invention relates to a stable particulate pharmaceutical composition obtained by using solifenacin or a salt thereof and a specific binder, a process for producing the same, a disintegrating tablet in buccal cavity comprising the particulate pharmaceutical composition, and a method of stabilizing the particulate pharmaceutical composition.

### Background Art

Solifenacin is represented by the following formula (I) : 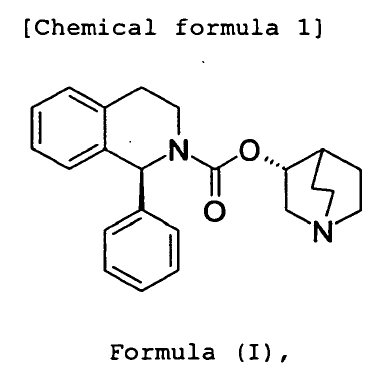 and its chemical name is (1R, 3'R) -3'-quinuclidinyl-1-phenyl-1,2,3,4-tetrahydro-2-isoquinoline carboxylate.

It has been reported that a series of quinuclidine derivatives including solifenacin and salts thereof have a highly selective antagonism to a muscarinic M₃ receptor, and is useful as a preventive/therapeutic agent for urologic diseases such as nervous pollakiuria, neurogenic bladder, nocturia, unstable bladder, bladder spasms and chronic cystitis or respiratory diseases such as chronic obstructive lung diseases, chronic bronchitis, asthma and rhinitis (see Patent document 1).

In Example 8 in the Patent document 1, a process for producing solifenacin hydrochloride is disclosed, and it is described that a crystal deposited in a mixed solvent composed of acetonitrile and diethyl ether had a melting point of 212 to 214°C and showed a specific rotation [α]²⁵_{D} of 98.1 (c = 1.00, EtOH).

However, in the Patent document 1, there is no description or even indication on an amorphous form of solifenacin or a salt thereof, or that when solifenacin succinate is formulated into a pharmaceutical preparation by a standard formulation method, the solifenacin succinate, which is an active ingredient, is significantly degraded with time in the produced pharmaceutical preparation.

In Non-patent document 1 issued by Ministry of Health, Labour and Welfare in June 2003, specification setting for pharmaceutical preparations, that is, concepts of degradation products (impurities) in pharmaceutical preparations accepted in a stability test is described. According to this document, in the case where the amount of a drug substance to be administered per day is less than 10 mg, the threshold for which the confirmation of the safety of the degradation products in the pharmaceutical preparation is required is the lower of the 1.0% in terms of the percentage of the degradation products contained in the drug substance and the 50 µg in terms of the total intake of the degradation products per day. In the case where the amount of a drug substance to be administered per day is 10 mg or more and 100 mg or less, the threshold for which the confirmation of the safety of the degradation products in the pharmaceutical preparation is required is the lower of the 0.5% in terms of the percentage of the degradation products contained in the drug substance and the 200 µg in terms of the total intake of the degradation products per day. Therefore, in general, the standard value of the amount of degradation products, which can be set without confirming the safety of the degradation products, is, for example, in the case of a pharmaceutical preparation in which the content of the drug substance is 5 mg, 1.0% or lower in terms of the percentage of the degradation products contained in the drug substance, and, for example, in the case of a pharmaceutical preparation in which the content of the drug substance is 10 mg, 0.5% or lower in terms of the percentage of the degradation products contained in the drug substance.

At present, pharmaceutical preparations of solifenacin, which are going to be sold on the market based on the results of the current clinical studies, are a 2.5 mg tablet, a 5 mg tablet and a 10 mg tablet. In order for such pharmaceutical preparations to have stability as described in the Non-patent document 1, it is considered that the amount of a major degradation product of solifenacin succinate (hereinafter referred to as F1) relative to the total amount of the solifenacin succinate and the degradation products has to be set to 0.5% or lower, and more preferably, there is a need to control it at 0.4% or lower including differences and errors among lots of the products and at the time of testing.

On the other hand, it is known that solifenacin and a salt thereof has very high solubility in various solvents and very strong bitterness and astringency. Therefore, in order to develop a pharmaceutical preparation with high convenience such as a particle or powder incorporated in a disintegrating tablet in buccal cavity of solifenacin or a salt thereof, there is a need to mask the bitterness and astringency. Thus, there was a need to apply a film-coating method using a polymeric substrate. More specifically, in the case where a drug substance is film coated with a polymeric substrate, there is a need to uniformly coat the surface of the drug substance. Thus, the drug substance had to be spherical fine particles with similar particle size.
[Patent document 1] EP Patent No.0801067
[Non Patent document 1] PFSB/ELD Notification No. 0624001 "Revision of the Guideline on the Impurities in the Medicinal Products with New Active Ingredients"

JP-A-2004-175796 discloses intraoral disintegrating preparations for treating dysuria. It mentions solifenacin succinate as one example of the drug to be incorporated into the preparation. A wide variety of excipients are also disclosed.

### Disclosure of the invention

### Problems that the Invention is to Solve

As described above, there was a need to provide a stable particulate pharmaceutical composition of solifenacin or a salt thereof, which is in a spherical shape suitable for film coating and in which degradation with time can be inhibited when a pharmaceutical preparation of solifenacin or a salt thereof is supplied to clinical fields.

### Means for Solving the Problems

Upon developing solifenacin succinate as an excellent therapeutic agent for frequent urination or urinary incontinence, the present inventors coated drug substances with a standard binder (polyvinylpyrrolidone (hereinafter abbreviated as PVP) or hydroxypropylmethyl cellulose (hereinafter abbreviated as HPMC)) , which a person skilled in the art generally conducts by the fluidized-bed granulation method or the like, and conducted a preliminary stability test for the resulting pharmaceutical preparations for over 2 months under accelerated test conditions (conditions of 40°C, 75% RH (relative humidity) and in an airtight bottle), which is one of the standard stability tests. As a result, a decrease in the residual ratio of solifenacin succinate was observed, and it was indicated that at 6 months after the initiation of storage, which is the time of the final determination in the accelerated test, the ratio of the production amount of F1 (the oxidized form of the solifenacin succinate) to the total amount of the solifenacin succinate and degradation products exceeds 0.4% (for more details, refer to the following Table 1). It was found that it is difficult to obtain a pharmaceutical preparation having a pharmaceutically sufficient stability by such a standard formulation method.

At such a technical level, the present inventors have made intensive studies for stabilizing a pharmaceutical preparation of solifenacin, and as a result, they found beyond expectation that solifenacin in an amorphous form formed in the production process of the pharmaceutical preparation is the major cause of degradation of the active ingredient with time, and the use of a standard binder such as HPMC is largely associated with the formation of the amorphous form of solifenacin.

To obtain a granular substance in which the bitterness and astringency of solifenacin are masked, the present inventor considered that a method in which a fine particle (particulate pharmaceutical composition) is prepared by spraying a solution of drug substance on a core particle composed of, for example, crystalline cellulose, and the fine particle is film coated with an appropriate polymeric substance is effective. To prepare such a fine particle, it is necessary to perform spraying after solifenacin or a salt thereof is dissolved once, however, it was found that solifenacin is liable to be amorphized at this time, and further, a problem specific to solifenacin that degradation products occur when it is converted from the amorphous form to the crystalline form arises. That is, in the case where a particulate pharmaceutical composition is produced after a part of or the whole of solifenacin is dissolved in a solvent, it was found that it is very difficult to ensure the stability of solifenacin.

Under such circumstances, the present inventors first found that when a substance having an ethylene oxide chain such as polyethylene glycol (another name: macrogol, hereinafter sometimes abbreviated as PEG) is used as a binder, a pharmaceutical preparation in which degradation of solifenacin with time can be inhibited by inhibiting the retention of an amorphous form of solifenacin is produced beyond expectation although PEG itself is a substance which is generally used for the purpose of amorphizing a drug substance.

Further, upon developing and producing a stable particulate pharmaceutical composition of solifenacin or a salt thereof suitable for film coating, the present inventors came up with the idea that, for example, in the case where dissolved solifenacin is sprayed on a core particle together with a polymeric substance (binder) such as PEG, whether or not the solifenacin can retain an amorphous form after the spraying may depend on the fluidity of solifenacin in the polymeric substance (binder). Therefore, they made intensive studies and paid attention on physical values (a glass transition point (hereinafter abbreviated as Tg) or a melting point (hereinafter abbreviated as mp)) specific to a polymer that may affect the fluidity of the drug substance as for a binder to be used in spraying the core particle. As a result, they found that when a binder having a high Tg was used for the particulate pharmaceutical composition, the initial value for a related substance, which becomes a degradation index, was low, however, as for the stability thereafter, it was unstable. On the other hand, when a specific binder having a Tg lower than a given value was used for the particulate pharmaceutical composition, they found beyond expectation that the initial value for a related substance and the value for a related substance generated thereafter were both low and stable, and moreover, the particle size was uniform and spherical, which is suitable for film coating.

Further, as a result of intensive studies, they found that when a crystallization-promoting treatment such as a humidification and drying treatment is performed, a more stable particulate pharmaceutical composition is produced, thus the present invention has been completed.

The present invention has the following embodiments.
1. A stable particulate pharmaceutical composition comprising:
   (i) solifenacin or a salt thereof, and
   (ii) a binder which stabilizes the solifenacin or salt thereof, the binder having a glass transition point or melting point lower than 174°C;
   wherein the binder is one or more of polyethylene glycol, polyethylene oxide, a polyoxyethylene/polyoxypropylene block copolymer, hydroxypropyl cellulose, hydroxyethyl cellulose, ethyl cellulose and maltose.
2. A pharmaceutical composition according to 1, wherein the binder is one or more of polyethylene glycol, a polyoxyethylene/polyoxypropylene block copolymer, hydroxypropyl cellulose, hydroxyethyl cellulose and maltose.
3. A pharmaceutical composition according to 2, wherein the binder is one or more of polyethylene glycol, a polyoxyethylene/polyoxypropylene block copolymer and hydroxypropyl cellulose.
4. A pharmaceutical composition according to any of 1-3, which is obtainable from a mixture in which the solifenacin or salt thereof and the binder are codissolved and/or suspended.
5. A pharmaceutical composition according to any of 1-4 which has been subjected to a crystallization-promoting treatment selected from a humidification treatment, a microwave irradiation treatment, an ultrasonic irradiation treatment, a low-frequency irradiation treatment and a thermal electron irradiation treatment in order to enhance its stability.
6. A tablet comprising a pharmaceutical composition according to any of 1-5, the tablet being capable of disintegrating in the buccal cavity.

In general, as an index of fluidity, thermodynamic parameters specific to a substance such as a glass transition point (Tg, unit: °C) or a softening point (unit: °C) based on an amorphous region in a polymer and a melting point (mp, unit: °C) based on a crystalline region in a polymer are used in many cases. These values indicate temperature showing a change in the thermodynamic state of a substance, however, because the molecular motion is suppressed at a temperature lower than Tg, the substance is in a state close to a crystalline state or a glass state, whereby the plasticity is liable to decrease. However, when the temperature of the substance is Tg or higher, the degree of the activity of the molecule increases, whereby the substance is in a rubber like state and the flexibility increases. Further, a state in which by an increase in the temperature, a crystalline region of a polymer is degraded to exhibit fluidity means melting of the polymer. By taking this into consideration, when a drug substance in an amorphous state exists in a polymer at a certain temperature, the higher the Tg of the polymer is, the more difficult the polymer itself flows. Therefore, it is liable to exist in an amorphous form, which is in an initial state, and on the contrary, it means that the lower the Tg of the polymer is, the sooner the crystal is deposited (Int. J. Pharm. 282 (2004) 151-162) . On the other hand, as for a low-molecular compound, because the structure of the substance is crystalline, it does not have a Tg in many cases, therefore, in terms of a parameter showing a change in the thermal mobility, a melting point was used as an index for a low-molecular compound. As for a polymer, Tg, which is a temperature at which the change appears faster, was used.

Solifenacin or a salt thereof can be stabilized by subjecting a pharmaceutical composition comprising the solifenacin or salt thereof and a binder as described above to a crystallization-promoting treatment.

An amorphous form of solifenacin or a salt thereof can be transferred to a crystalline form by subjecting a stable particulate composition comprising the solifenacin or salt thereof and a binder as described above to a crystallization-promoting treatment.

Hereinafter, the composition of the present invention will be described in detail.

Examples of the "salt of solifenacin" to be used in the present invention include solifenacin hydrochloride described in the Patent document 1, acid addition salts with mineral acids such as hydrobromic acid, hydriodic acid, sulfuric acid, nitric acid and phosphoric acid or with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid and glutamic acid, and quaternary ammonium salts. Among these, solifenacin succinate is preferred in consideration of being provided as a pharmaceutical product.

The "solifenacin or a salt thereof" to be used in the present invention can be easily obtained by or in accordance with the method described in the Patent document 1 or by a standard method.

The "crystal" or the "crystalline form" of solifenacin or a salt thereof means a literal interpretation of a substance of solifenacin or a salt thereof having a crystallographically crystalline structure. However, in the present invention, it means a substance different from an "amorphous form" which shows significant degradability with time of solifenacin when it is contained in an amount within the range which exerts no influence on the stability of the product in the pharmaceutical preparation. On the other hand, the "amorphism" or the "amorphous form" of solifenacin or a salt thereof in the present invention means a substance having a crystallographically amorphous structure. However, in the present invention, it means a substance different from a "crystal" or a "crystalline form" which shows extremely little degradability with time of solifenacin when it is contained in an amount exceeding the range which exerts no influence on the stability of the product in the pharmaceutical preparation.

The mixing amount of solifenacin or a salt thereof to be used in the present invention is generally selected suitably according to the type of drug substance or the medicinal use thereof (indication), however, it is not particularly limited as long as it is a therapeutically effective amount or a prophylactically effective amount. Specifically, it is from 0.01 mg to 100 mg, preferably from 0.5 mg to 50 mg, more preferably from 0.5 mg to 10 mg, and most preferably from 0.5 mg to 4 mg in terms of the daily amount of solifenacin or a salt thereof.

Further, the mixing amount of solifenacin or a salt thereof in the disintegrating tablet in buccal cavity of the present invention may be any as long as an effective amount per administration unit of the pharmaceutical preparation is contained, however, it is preferably from 0.001% by weight to 97% by weight, more preferably from 0.05% by weight to 50% by weight, further more preferably from 0.05% by weight to 10% by weight, and most preferably from 0.05% by weight to 4% by weight.

The "binder having an action of stabilizing solifenacin or a salt thereof" to be used in the present invention means a binder that can inhibit degradation with time of solifenacin or a salt thereof, and specifically means a binder that can inhibit degradation with time of solifenacin or a salt thereof by the action of inhibiting retention of an amorphous form. In addition, in the case where a binder that does not have an action of stabilizing solifenacin or a salt thereof alone, for example, even a binder such as HPMC or PVP, is used together with the binder to be used in the present invention for the purpose of enhancing the action as a binder, it can be used in an amount within the range which does not exceed the specification setting of the stability of the pharmaceutical preparation, which is an object of the present invention.

The "action of inhibiting retention of an amorphous form" as used in the present invention refers to an action of making the compound difficult to exist in an amorphous state and/or an action capable of making the compound easy to be transformed from the amorphous form to the crystalline form.

Further, the binder having an action of stabilizing solifenacin or a salt thereof or an action of inhibiting retention of an amorphous form to be used in the present invention is a binder capable of reducing the amount of F1 of solifenacin to 0.5% or lower, more preferably a binder capable of reducing the amount of F1 to 0.4% or lower. Specifically, it is a binder having a Tg or mp lower than 174°C, preferably a binder having a Tg or mp of 0°C or higher and lower than 174°C, more preferably a binder having a Tg or mp of 0°C or higher and lower than 156°C, further more preferably a binder having a Tg or mp of 0°C or higher and lower than 137°C, and most preferably a binder having a Tg or mp of 10°C or higher and lower than 137°C.

The binder is preferably PEG, polyethylene oxide, a polyoxyethylene/polyoxypropylene block copolymer, hydroxypropyl cellulose, hydroxyethyl cellulose or maltose, more preferably PEG, a polyoxyethylene/polyoxypropylene block copolymer or hydroxypropyl cellulose, particularly preferably PEG or hydroxypropyl cellulose, and most preferably PEG for case of coating of a particle. The molecular weight and the degree of polymerization of the binder are not particularly limited as long as the object of the present invention to inhibit the amorphization of solifenacin or a salt thereof can be attained by the addition of the binder. However, the weight average molecular weight is preferably within the range from 400 to 1,000,000, and more preferably the weight average molecular weight is within the range from 2,000 to 200,000. In addition, the binders described above can be used in combination of two or more types.

The substances having an ethylene oxide chain may be used alone or by mixing two or more types. Among these, PEG and a polyoxyethylene/polyoxypropylene block copolymer are preferred, and PEG is particularly preferred. As the PEG, PEG in a solid form at normal temperature is preferred. Specific examples include Macrogol 4000 (Japanese Pharmacopoiea, molecular weight: from 2,600 to 3,800, brand name: Macrogol 4000/ Sanyo Chemical Industries, Ltd., NOF Corporation, Lion Corporation, and the like), Macrogol 6000 (Japan Pharmaceopoiea, molecular weight: from 7,300 to 9,300, brand name: Macrogol 6000/ Sanyo Chemical Industries, Ltd., NOF Corporation, Lion Corporation, and the like), Macrogol 20000 (Japan Pharmacopoeia, molecular weight: from 15, 000 to 25, 000, brand name: Macrogol 20000/ Sanyo Chemical Industries, Ltd., NOF Corporation, Lion Corporation, and the like), polyethylene glycol 8000 (USP/ NF, molecular weight: from 7,000 to 9,000, brand name: Polyethylene glycol 8000/ The Dow Chemical Company, and the like), and the like. The weight average molecular weight of PEG is preferably within the range from 400 to 40, 000, more preferably within the range from 2,000 to 25,000, and further more preferably within the range from 2, 000 to 10, 000.

The polyoxyethylene/polyoxypropylene block copolymer of the present invention is a copolymer of propylene oxide and ethylene oxide, and various types exist depending on the composition ratio thereof, however, it may has a composition ratio so as to have a property of inhibiting the amorphization of solifenacin or a salt thereof. Specifically, polyoxyethylene (105) polyoxypropylene (5) glycol, polyoxyethylene (160) polyoxypropylene (30) glycol (another name: Pluronic F68) or the like is used.

The "mixture in which solifenacin or a salt thereof and a binder having an action of stabilizing solifenacin or a salt thereof are codissolved and/or suspended" as used in the present invention means a mixture in which a binder having an action of stabilizing solifenacin or a salt thereof is dissolved together with a solution obtained by dissolving solifenacin or a salt thereof in a solvent such as water. However, it is not always necessary that the whole of solifenacin or a salt thereof be dissolved in a solvent, and as long as a particle containing a drug substance suitable for coating such as masking of bitterness, which is performed thereafter, can be obtained with the resulting mixture, such a mixture to given a particle obtained by using the mixture in a suspended state in which a part of solifenacin or a salt thereof is dissolved in a solvent is also included.

The composition, which "can be obtained by using" a mixture in which solifenacin or a salt thereof and a binder having an action of stabilizing solifenacin or a salt thereof are codissolved, in the present invention is a particle containing a drug substance suitable for coating such as masking of bitterness. Examples thereof include a composition obtained by spray coating a core particle of such as crystalline cellulose with a drug substance in a solution form, a composition that can be obtained not by spraying a drug substance in a liquid form, but by mixing a mixture obtained by codissolving these substances with an insoluble core particle and depositing the drug substance to arrange the drug substance uniformly around the insoluble core particle, and the like. Further, examples of a product prepared by a method without using a core particle include a powder itself obtained by spray-drying or freeze-drying a solution of a drug substance and a binder, and such a powder can be used for a particle containing a drug substance to be used for masking bitterness or the like. However, in view of the production efficiency, a composition that can be obtained by spray-coating a core particle with a mixture in which solifenacin or a salt thereof and a binder having an action of stabilizing solifenacin or a salt thereof are codissolved is preferred.

The "stable particulate pharmaceutical composition" as used in the present invention is a particle that can be obtained by using solifenacin or a salt thereof or the like, and is not particularly limited as long as it is a stable particle in which degradation with time is inhibited. The term "stable" as used herein specifically means a particle in which the production amount of F1 of solifenacin or a salt thereof is 0.5% or lower, more preferably 0.4% or lower. Further; in the case where the pharmaceutical composition of the present invention is a particle such as a granule, the particle size of the particulate pharmaceutical composition is not particularly limited as long as the longest diameter is 2 mm or less. As for the case where it is incorporated in a disintegrating tablet in buccal cavity, the particle size is not particularly limited as long as there is not an unpleasant gritty sensation like sand when it is taken, however, it is preferably prepared at an average particle size of 350 µm or less. The more preferred average particle size is from 1 to 350 µm, and the particularly preferred average particle size is from 20 to 350 µm. In view of the particle size distribution, it is not particularly limited as long as it is a particle suitable for coating such as masking of bitterness, however, preferably, 80% of the total weight is distributed between 1 and 350 µm, more preferably 80% of the total weight is distributed between 50 and 300 µm, and particularly preferably 80% of the total weight is distributed between 100 and 250 µm.

Further, the shape of the particulate composition of the present invention is not particularly limited as long as it is in a state where coating such as masking of bitterness can be performed, however, in terms of the coating efficiency, it is preferably in a spherical shape, that is, the sphericity thereof is preferably as close to 1 as possible.

In the case where the particulate pharmaceutical composition of the present invention is a granule, the mixing amount of the binder in the particulate pharmaceutical composition is not particularly limited as long as it is an amount that enables the coating with solifenacin or a salt thereof and attains the object of the present invention. However, it is preferably from 0.01 to 91% by weight, and more preferably from 0.5 to 75% by weight of the total particulate pharmaceutical composition. The most preferred mixing amount is from 5 to 50% by weight. Further, by taking the mixing amount of the binder in the case where the pharmaceutical composition of the present invention is a particle such as a granule into consideration relative to the 1 part by weight of solifenacin or a salt thereof in a crystalline form and an amorphous form, it is preferably at a ratio ranging from 1 to 1, 000% by weight, more preferably at a ratio ranging from 5 to 500% by weight, and further more preferably at a ratio ranging from 10 to 100% by weight.

The particulate pharmaceutical composition of the present invention is prepared with solifenacin or a salt thereof in a solution state. However, in the case where a core particle is spray coated with a solifenacin solution, examples of the core particle include sodium chloride, microcrystalline cellulose, calcium carbonate, lactose, maltose and mannitol, and preferred examples thereof include microcrystalline cellulose, lactose, mannitol and the like. More preferred are microcrystalline cellulose and lactose. In the present invention, among a group of these substances, one type or two or more types can be used in combination.

Further, the crystallization-promoting treatment as used herein is not particularly limited as long as it is a treatment of promoting crystallization, and examples thereof include a humidification treatment, a microwave irradiation treatment, an ultrasonic irradiation treatment, a low-frequency irradiation treatment, a thermal electron irradiation treatment and the like. Further, the humidification treatment refers to a treatment in which, for example, a humidification treatment at a temperature of from 20 to 30°C and at a humidity of from 60 to 85% RH for 6 to 24 hours is carried out, and then drying at a temperature of from 30 to 40°C and at a humidity of from 30 to 40% RH for 2 to 6 hours is carried out. The microwave irradiation treatment cannot be limited in general, however, for example, a microwave with a wavelength of from 10 MHz to 25 GHz can be used. Further, the treatment time depends on the degree of crystallization at an initial stage and a selected base material, however, for example, the treatment can be carried out for 10 seconds to 60 minutes. The irradiation per se may be carried out continuously or intermittently, and at any timing after any particulate composition is produced.

The ultrasonic irradiation treatment cannot be limited in general, however, for example, an ultrasonic wave with a frequency of from 10 kHz to 600 kHz can be used. Further, the treatment time depends on the degree of crystallization at an initial stage and a selected base material, however, for example, the treatment can be carried out for 10 seconds to 24 hours. The irradiation per se may be carried out continuously or intermittently, and at any timing after any particulate composition is produced.

As the crystallization-promoting treatment, a humidification treatment, a microwave irradiation treatment and an ultrasonic irradiation treatment are preferred.

In the particulate pharmaceutical composition of the present invention, any of a variety of pharmaceutical excipients is suitably used and formulated into a pharmaceutical preparation. Examples of such a pharmaceutical excipient include lactose and the like. Further, another additive can be used within the range that does not impair the object of the present invention as long as it is a pharmaceutically and pharmacologically acceptable one. For example, a disintegrating agent, an acidifier, a foaming agent, an artificial sweetener, a flavor, a lubricant, a coloring agent, a stabilizing agent, a buffering agent, an antioxidant, a surfactant or the like can be used, and there is no particular limitation. Examples of the disintegrating agent include cornstarch, potato starch, carmellose calcium, carmellose sodium, low-substitution degree hydroxypropyl cellulose and the like. Examples of the acidifier include citric acid, tartaric acid, malic acid and the like. Examples of the foaming agent include sodium bicarbonate and the like. Examples of the artificial sweetener include saccharin sodium, dipotassium glycyrrhizinate, aspartame, stevia, somatin and the like. Examples of the flavor include lemon, lemon-lime, orange, menthol and the like. Examples of the lubricant include magnesium stearate, calcium stearate, a sucrose fatty acid ester, talc, stearic acid and the like. Examples of the coloring agent include yellow iron sesquioxide, red iron sesquioxide, food yellow No. 4 and No. 5, food red No. 3 and No. 102, food blue No. 3 and the like. Examples of the buffering agent include citric acid, succinic acid, fumaric acid, tartaric acid, ascorbic acid or a salt thereof, glutamic acid, glutamine, glycine, asparatic acid, alanine, arginine, or a salt thereof, magnesium oxide, zinc oxide, magnesium hydroxide, phosphoric acid, boric acid or a salt thereof and the like. Examples of the antioxidant include ascorbic acid, sodium nitrite, sodium sulfite, sodium hydrogen sulfite, sodium edetate, erythorbic acid, tocopherol acetate, tocopherol, butylhydroxyanisol, dibutylhydroxytoluene, propyl gallate and the like. Examples of the surfactant include sodium lauryl sulfate, a polyoxyethylene sorbitan fatty acid ester (polysorbate 80), polyoxyethylene hydrogenated castor oil and the like. As for the pharmaceutical excipient, the above-mentioned substances can be suitably added alone or in combination of two or more types in an appropriate amount.

The "content of the amorphous form" as used in the present invention means the ratio relative to the total of the amorphous form and the crystalline form of solifenacin or a salt thereof.

Examples of the pharmaceutical preparation using the above-mentioned particle include a powder, a granule, a pill, a tablet, a capsule, an disintegrating tablet in buccal cavity, a dry syrup and the like, however, particularly, an disintegrating tablet in buccal cavity is preferred.

Hereinafter, a disintegrating tablet in buccal cavity containing the particulate pharmaceutical composition of the present invention will be described.

The "disintegrating tablet in buccal cavity" in the present invention means a tablet which is disintegrated in the buccal cavity with substantially saliva only within 2 minutes, preferably within 1 minute, more preferably within 30 seconds in the case where the tablet is taken without having any water, and a pharmaceutical preparation similar to the tablet.

The particulate pharmaceutical composition of the present invention can be incorporated in such a disintegrating tablet in buccal cavity, for example, a disintegrating tablet in buccal cavity can be prepared by applying the particulate pharmaceutical composition as the drug substance of a known disintegrating tablet in buccal cavity described in any of International Publication No. 95-20380 (corresponding to US Patent No. 5576014), International Publication No. 2002-92057 (corresponding to US Patent Application Publication No. 2003/099701), US Patent No. 4305502, US Patent No. 4371516, Japanese Patent No. 2807346 (corresponding to US Patent No. 5466464), JP-A-5-271054 (corresponding to European Patent No. 553777), JP-A-10-182436 (corresponding to US Patent No. 5958453), Japanese Patent No. 3412694 (corresponding to US Patent No. 5223264) and International Publication No. WO 98/02185 (corresponding to US Patent No. 6287596), using the base material of a disintegrating tablet in buccal cavity described in any of the publications, and following the method described in any of the publications. In this way, as the disintegrating tablet in buccal cavity containing the particulate pharmaceutical composition, the disintegrating tablets in buccal cavity described in Japanese Patent No. 3412694 (corresponding to US Patent No. 5223264) and JP-A-2003-55197 can be exemplified, and the particulate pharmaceutical composition of the present invention can be incorporated in such a disintegrating tablet in buccal cavity.

In general, the disintegrating tablet in buccal cavity as illustrated above is classified roughly into a mold type, a humidified type and a standard tablet type, and the particulate pharmaceutical composition of the present invention may be incorporated in any type of disintegrating tablet in buccal cavity. The mold type of disintegrating tablet in buccal cavity is prepared, for example, by filling a mold with a solution or suspension of such as an excipient and drying it as disclosed in Japanese Patent No. 2807346 (corresponding to US Patent No. 5466464). The mold type of disintegrating tablet in buccal cavity containing the particulate pharmaceutical composition of the present invention can be prepared, for example, by filling a blister package with a solution or suspension of the particulate pharmaceutical composition of the present invention, an excipient such as a saccharide and a binder such as gelatin or agar and removing moisture by a method such as freeze-drying, reduced-pressure drying or low-temperature drying. The humidified type of disintegrating tablet in buccal cavity is prepared by humidifying an excipient such as a saccharide, and performing tableting at a low pressure and then drying it as described in Japanese Patent No. 3069458 (corresponding to US Patent No. 5501861 and US Patent No. 5720974) . Therefore, for example, the particulate pharmaceutical composition of the present invention and an excipient such as a saccharide are humidified with a small amount of water or a mixed solution of water and an alcohol, and the resulting humidified mixture is molded at a low pressure, and then drying the molded mixture, whereby the humidified type of disintegrating tablet in buccal cavity can be prepared.

In the case of the standard tablet type, it is prepared through a standard tableting step as disclosed in International Publication No. 95-20380 (corresponding to US Patent No. 5576014), International Publication No. 2002-92057 (corresponding to US Patent Application Publication No. 2003/099701), JP-A-10-182436 (corresponding to US Patent No. 5958453), JP-A-9-48726, JP-A-8-19589 (corresponding to US Patent No. 5672364), Japanese Patent No. 2919771, and Japanese Patent No. 3069458 (corresponding to US Patent No. 5501861 and US Patent No. 5720974) . In order to prepare the standard tablet type of disintegrating tablet in buccal cavity containing the particulate pharmaceutical composition of the present invention, for example, the particulate pharmaceutical composition of the present invention and an excipient such as a saccharide with low moldability are granulated by using a solution or suspension of a saccharide with high moldability or a water-soluble polymer, and then the resulting granulated substance is compression molded to form a compression-molded substance, or further the resulting compression-molded substance is subjected to humidification and drying, whereby the disintegrating tablet in buccal cavity can be prepared as disclosed in International Publication No. 95-20380 (corresponding to US Patent No. 5576014) and Japanese Patent No. 2919771. Further, in order to prepare the standard tablet type of disintegrating tablet in buccal cavity as shown in International Publication No. 99-47124 (corresponding to US Patent No. 6589554), for example, the particulate pharmaceutical composition of the present invention and an excipient such as a crystalline saccharide are compression molded by using an amorphous saccharide, and the resulting substance is subjected to humidification and drying, whereby the disintegrating tablet in buccal cavity can be prepared. Further, in order to prepare the standard tablet type of disintegrating tablet in buccal cavity as disclosed in International Publication No. 2002-92057 (corresponding to US Patent Application Publication No. 2003/099701), for example, a mixture of the particulate pharmaceutical composition of the present invention and an excipient with a saccharide with a melting point lower than that of the excipient is compression molded, and the resulting substance is heated to form a cross-linkage by the melt-solidified product of the saccharide with a lower melting point, whereby the disintegrating tablet in buccal cavity can be prepared. By the humidification and drying or the heating treatment as described above, the tablet strength of such a disintegrating tablet in buccal cavity can be improved.

As the excipient to be used in the disintegrating tablet in buccal cavity of the present invention, a standard excipient can also be used, however, particularly, a pharmaceutically acceptable saccharide is preferably used. In a technique utilizing the moldability of a saccharide, a saccharide with low moldability can be used. When a technique for improving the tablet strength by the crystalline/amorphous property of a saccharide and humidification and drying is used, a crystalline saccharide can be used. When a technique for forming a cross-linkage by the melt-solidified product of a saccharide is used, a saccharide with a high melting point can be used in addition to a standard excipient.

The "saccharide with low moldability" means a saccharide that provides a tablet hardness of from 0 to 2 kp when, for example, 150 mg of the saccharide is tableted at a tableting pressure of from 10 to 50 kg/cm² using a punch with a diameter of 8 mm. The "saccharide with high moldability" means a saccharide that provides a tablet hardness of 2 kp or greater by the same method. The saccharide with low moldability is a pharmaceutically acceptable one, and examples thereof may include lactose, mannitol, glucose, sucrose, xylitol, erythritol and the like. These saccharides can be used alone or by suitably combining two or more types. The saccharide with high moldability is a pharmaceutically acceptable one, and examples thereof may include maltose, maltitol, sorbitol, trehalose and the like. Also, these saccharides can be used alone or by suitably combining two or more types.

The "crystalline saccharide" is a pharmaceutically acceptable one, and examples thereof may include mannitol, maltitol, erythritol, xylitol and the like. These saccharides can be used alone or by suitably combining two or more types. The "amorphous saccharide" is a pharmaceutically acceptable one, and examples thereof may include lactose, sucrose, glucose, sorbitol, maltose, trehalose and the like. Also, these saccharides can be used alone or by suitably combining two or more types.

Further, an "excipient with a melting point higher than that of a saccharide with a low melting point" is a pharmaceutically acceptable one, and can be selected from, for example, xylitol, trehalose, maltose, sorbitol, erythritol, glucose, sucrose, maltitol, mannitol and the like. These saccharides can be used alone or by suitably combining two or more types. The "saccharide with a low melting point" is a pharmaceutically acceptable one, and can be selected from, for example, xylitol, trehalose, maltose, sorbitol, erythritol, glucose, sucrose, maltitol, mannitol and the like. Also, these saccharides can be used alone or by suitably combining two or more types. As the binder for the disintegrating tablet in buccal cavity, maltitol, copolyvidone, erythritol and the like can be exemplified. Also, these binders can be used alone or by suitably combining two or more types.

When a water-soluble polymer is used in place of a saccharide with high moldability, preferred are, for example, hydroxypropyl cellulose, hydroxypropylmethyl cellulose, povidone, polyvinyl alcohol, gum arabic powder, gelatin, purulan, and the like.

The mixing amount of the excipient to be used in the disintegrating tablet in buccal cavity containing the particulate pharmaceutical composition of the present invention is suitably adjusted according to the mixing amount of the particulate pharmaceutical composition of the present invention and/or the size of the tablet and the like, however, it is preferably from 20 to 1000 mg in general, more preferably from 50 to 900 mg and particularly preferably from 100 to 800 mg per tablet.

Further, the mixing amount of the saccharide with high moldability, the water-soluble polymer, the amorphous saccharide, or the saccharide with a low melting point is not particularly limited as long as is it suitably selected and used according to the respective techniques, however, it is preferably from 0.5 to 40% by weight, more preferably from 2 to 30% by weight, and particularly from 5 to 20% by weight of the weight of the excipient, or it is preferably from 1 to 20% by weight of the total pharmaceutical preparation.

As for the type of another optional additive, the formulation or the mixing amount thereof, or the like, the description of the above-mentioned patent documents for the disintegrating tablets in buccal cavity is cited as the description of this specification.

Further, in the case where the particulate pharmaceutical composition of the present invention is incorporated in the disintegrating tablet in buccal cavity, the particulate pharmaceutical composition can be incorporated in an amount corresponding to 0.5 to 90% by weight, preferably 1 to 80% by weight, and more preferably 5 to 60% by weight of the total disintegrating tablet in buccal cavity.

Hereinafter, a process for producing the particulate pharmaceutical composition of the present invention will be described.

In order to obtain the particulate pharmaceutical composition of the present invention, solifenacin or a salt thereof and a binder having an action of stabilizing solifenacin or a salt thereof are dissolved or suspended by agitation in water or a mixed solution obtained by adding an organic solvent such as ethanol to water using a stirrer, whereby a drug substance solution is prepared. In this case, water or the organic solvent contained in the drug substance solution can be appropriately set. As a technique for powderization (granulation) of the drug substance solution of solifenacin or a salt thereof, for example, a freeze-drying method, a spray-drying method, a high-shared agitation granulation method, a fluidized bed granulation method, a tumbling granulation method and the like can be exemplified. As long as the method enables the powderization (granulation) of solifenacin after it is dissolved, both the device and the technique are not particularly limited, however, particularly preferred are a spray-drying method and a fluidized bed granulation method. Specifically, an appropriate additive particle (for example, crystalline cellulose (particle), a purified sucrose spherical granule, a sucrose starch spherical granule or the like) to be a core is spray coated with the drug substance solution, whereby the particulate composition of the present invention can be obtained.

As the device to be used upon the spray coating, for example, a fluidized bed granulator (FLO-1, manufactured by Glatt Co., Ltd.), a spray dryer (DL41, manufactured by Yamato Scientific Co. Ltd.) and the like can be exemplified.

Hereinafter, a process for producing the disintegrating tablet in buccal cavity containing the particulate pharmaceutical composition of the present invention will be described.

When the case of the disintegrating tablet in buccal cavity described in International Publication No. 95-20380 (corresponding to US Patent No. 5576014) is cited, the steps of mixing the particulate pharmaceutical composition of the present invention and a saccharide with low moldability; spraying the resulting mixture using a saccharide with high moldability as a binder to perform coating and/or granulation; and subjecting the resulting granulated matter to compression molding can be adopted. Further, in order to heighten the hardness of the prepared molded matter, humidification and drying steps can be adopted. The "humidification" is determined by the apparent critical relative humidity of the saccharide to be contained, but it is humidified generally to the critical relative humidity or higher. For example, the humidity is from 30 to 100 RH %, preferably from 50 to 90 RH %. In this case, the temperature is preferably from 15 to 50°C, more preferably from 20 to 40°C. The treating time is from 1 to 36 hours, preferably from 12 to 24 hours. The "drying" is not particularly limited as long as it is a step of removing moisture absorbed by the humidification. For example, as a drying temperature condition, it can be set to 10 to 100°C, preferably 20 to 60°C, more preferably 25 to 40°C. The treating time can be set to 0.5 to 6 hours, preferably 1 to 4 hours.

In the case of the disintegrating tablet in buccal cavity described in International Publication No. 2002-92057 (corresponding to US Patent Application Publication No. 2003/099701), the particulate pharmaceutical composition of the present invention, an excipient with a high melting point and a saccharide with a low melting point are mixed, and the resulting mixture is sprayed by using a binder for disintegrating tablet in buccal cavity to perform coating and/or granulation, and then, the granulated matter can be subjected to compression molding. As the spraying condition, for example, when a fluidized bed granulator (FLO-1, manufactured by Glatt Co., Ltd.) is used, the concentration of the solution of solifenacin is not limited as long as it gives a viscosity capable of sending the solution with a pump, however, it is preferably from 0.01 to 30% (w/w) in terms of the solid content concentration. The spraying rate is not limited as long as spray drying can be done, however, it is preferably from 0.1 to 20 g/min. The spraying temperature is not particularly limited as long as it can be set so as to give a product temperature of from 10 to 60°C. These spraying conditions vary depending on the production scale and the type of device, however, they are not particularly limited as long as they provide a particulate composition. Further, in the case where an excipient with a high melting point and a saccharide with a low melting point are combined, in order to heighten the hardness of the prepared molded matter, a heating step can be adopted. The "heating" is determined by the melting point of the saccharide with a low melting point contained therein. However, it is generally heated to a temperature not lower than the melting point of the saccharide with a low melting point and lower than the melting point of the excipient with a high melting point. The treating time can be set to 0.5 to 120 minutes, and preferably 1 to 60 minutes.

Further, the method of stabilizing the particulate pharmaceutical composition of the present invention and the method of converting an amorphous form of solifenacin or a salt thereof to a crystalline form in the particulate pharmaceutical composition of the present invention can be carried out for the particulate composition of the present invention produced as described above by using the above-mentioned crystallization-promoting treatment method.

### Brief Description of the Drawings

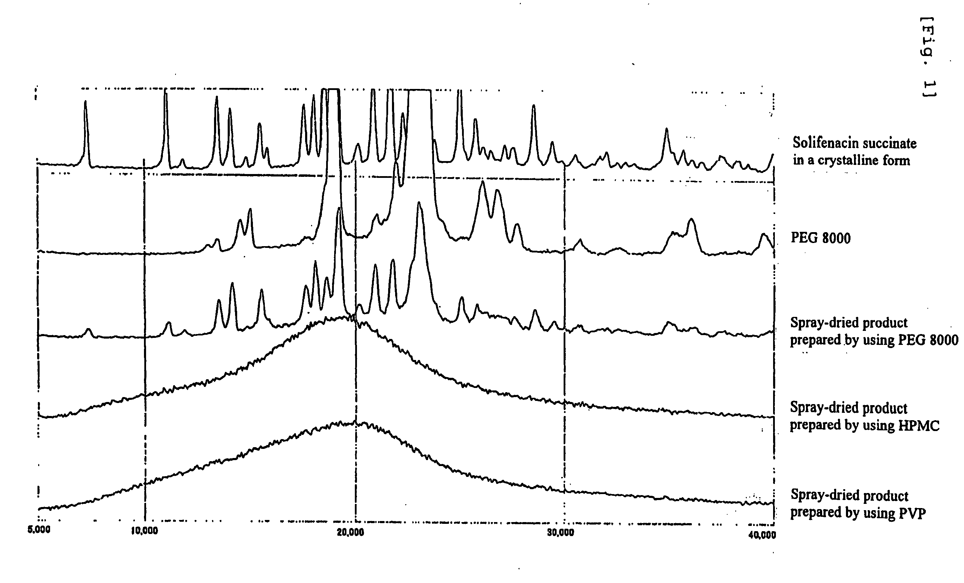

[Fig. 1] Fig. 1 shows the results of powder X-ray diffraction for solifenacin succinate in a crystalline form; PEG 8000 (brand name: Macrogol 6000, manufactured by Sanyo Chemical Industries, Ltd.); a spray-dried product of solifenacin succinate prepared by using PEG 8000; a spray-dried product of solifenacin succinate prepared by using HPMC (brand name: TC-5E, manufactured by Shinetsu Chemical Co., Ltd.) ; and a spray-dried product of solifenacin succinate prepared by using polyvinylpyrrolidone (brand name: PVP K90, manufactured by BASF, hereinafter abbreviated as PVP), respectively.

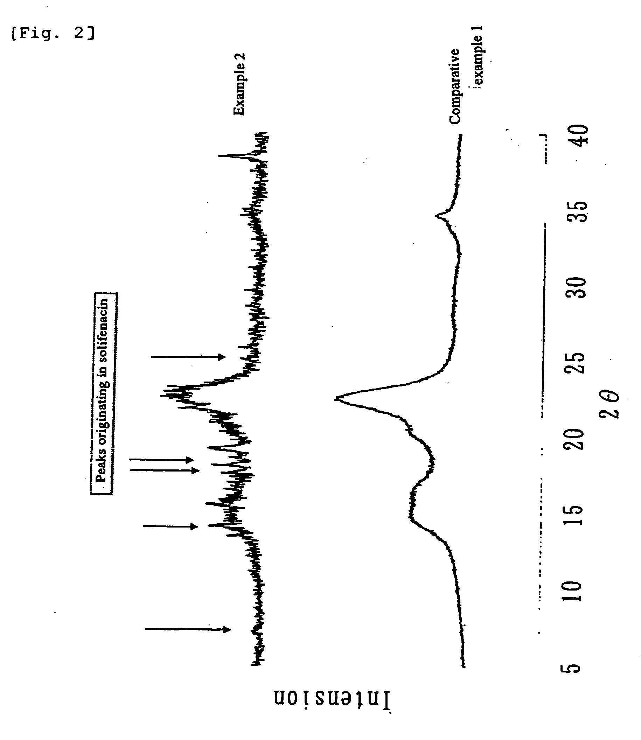

[Fig. 2] Fig. 2 shows the results of powder X-ray diffraction for a coated product obtained by coating crystalline cellulose (brand name: Celphere, manufactured by Asahi Chemical Industry Co., Ltd.) with PEG 8000 (brand name: Macrogol 6000, manufactured by Sanyo Chemical Industries, Ltd.) in Example 3; and a coated product obtained by using HPMC in Comparative example 1.

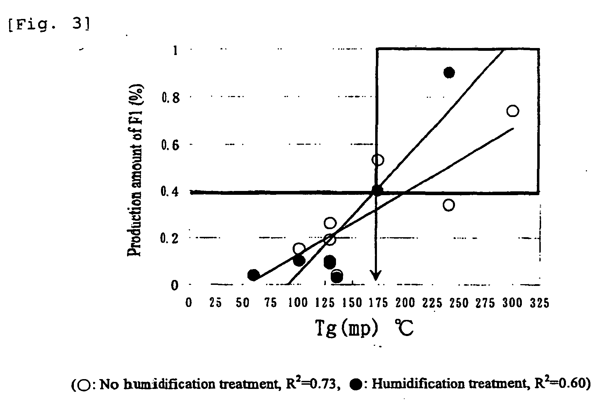

[Fig. 3] Fig. 3 shows the relationship between the production amount of F1, which is a major degradation product of solifenacin, and the Tg or mp of a binder used in combination after a two-month storing period (O: without humidification treatment, ●: with humidification treatment).

### Best Mode for Carrying Out the Invention

A particulate pharmaceutical composition of solifenacin or a salt thereof in the present invention will be described in detail. Hereinafter, the present invention will be described in more detail with reference to Examples and Comparative examples, however, the present invention is not construed as being limited to these.

### Example 1

### A coated product obtained by coating a crystalline cellulose core particle with solifenacin succinate using HPC-SL as a binder

Ten parts of solifenacin succinate and 3.4 parts of hydroxypropyl cellulose (brand name: HPC-SL, manufactured by Nippon Soda Co., Ltd., hereinafter abbreviated as HPC) were dissolved by agitation in a mixed solution of 26.6 parts of water and 26.6 parts of methanol using a stirrer (MGM-66, manufactured by SHIBATA), whereby a drug substance solution was prepared. Then, 60 parts of crystalline cellulose (brand name: Celphere, manufactured by Asahi Chemical Industry Co., Ltd.) were put into a fluidized bed granulator (FLO-1, manufactured by Glatt Co., Ltd.), and Celphere was spray coated with the drug substance solution at an intake air temperature of 50°C, an air flow volume of 1.00 m³/min, a binder solution-spraying rate of 4.0 g/min, and a spraying air pressure of 3.0 kg/cm², whereby a particulate composition of the present invention was obtained.

### Example 2

The particulate composition obtained in Example 1 was subjected to a crystallization treatment by humidification at 25°C and 75% for 12 hours, and then drying at 30°C and 40% for 3 hours, whereby a particulate composition of the present invention was obtained.

### Example 3

### A coated product obtained by coating a crystalline cellulose core particle with solifenacin succinate using PEG 6000 as a binder

Ten parts of solifenacin succinate and 3.4 parts of PEG (brand name: Macrogol 6000, manufactured by Sanyo Chemical Industries, Ltd.) were dissolved by agitation in a mixed solution of 26. 6 parts of water and 26. 6 parts of methanol using a stirrer (MGM-66, manufactured by SHIBATA), whereby a drug substance solution was prepared. Then, 60 parts of Celphere (manufactured by Asahi Chemical Industry Co., Ltd.) were put into a fluidized bed granulator (FLO-1, manufactured by Glatt Co., Ltd.), and Celphere was spray coated with the drug substance solution at an intake air temperature of 50°C, an air flow volume of 0. 97 m³/min, a binder solution-spraying rate of 10 g/min, and a spraying air pressure of 3.0 kg/cm², whereby a particulate composition of the present invention was obtained.

### Example 4

The particulate composition obtained in Example 3 was subjected to a crystallization treatment by humidification at 25°C and 75% for 12 hours, and then drying at 30°C and 40% for 3 hours, whereby a particulate composition of the present invention was obtained.

### Example 5

### A coated product obtained by coating a crystalline cellulose core particle with solifenacin succinate using maltose as a binder

Ten parts of solifenacin succinate and 3.4 parts of maltose (brand name: SunMalto S, manufactured by Sanwa Cornstarch Co., Ltd.) were dissolved by agitation in a mixed solution of 26. 6 parts of water and 26. 6 parts of methanol using a stirrer (MGM-66, manufactured by SHIBATA), whereby a drug substance solution was prepared. Then, 60 parts of Celphere (manufactured by Asahi Chemical Industry Co., Ltd.) were put into a fluidized bed granulator (FLO-1, manufactured by Glatt Co., Ltd.), and Celphere was spray coated with the drug substance solution at an intake air temperature of 60°C, an air flow volume of 0.98 m³/min, a binder solution-spraying rate of 3. 0 g/min, and a spraying air pressure of 3. 0 kg/cm², whereby a particulate composition of the present invention was obtained.

### Example 6

The particulate composition obtained in Example 5 was subjected to a crystallization treatment by humidification at 25°C and 75% for 12 hours, and then drying at 30°C and 40% for 3 hours, whereby a particulate composition of the present invention was obtained.

### Example 7

### A coated product obtained by coating a crystalline cellulose core particle with solifenacin succinate using HEC as a binder

Ten parts of solifenacin succinate and 3.4 parts of hydroxyethyl cellulose (brand name: HEC SE400, manufactured by Daicel Chemical Industries, Ltd.) were dissolved by agitation in a mixed solution of 26.6 parts of water and 26.6 parts of methanol using a stirrer (MGM-66, manufactured by SHIBATA), whereby a drug substance solution was prepared. Then, 60 parts of Celphere (manufactured by Asahi Chemical Industry Co., Ltd.) were put into a fluidized bed granulator (FLO-1, manufactured by Glatt Co., Ltd.), and Celphere was spray coated with the drug substance solution at an intake air temperature of 60°C, an air flow volume of 0.98 m³/min, a binder solution-spraying rate of 3.0 g/min, and a spraying air pressure of 3.0 kg/cm², whereby a particulate composition of the present invention was obtained.

### Example 8

The particulate composition obtained in Example 7 was subjected to a crystallization treatment by humidification at 25°C and 75% for 12 hours, and then drying at 30°C and 40% for 3 hours, whereby a particulate composition of the present invention was obtained.

### Example 9

### A coated product obtained by coating a crystalline cellulose core particle with solifenacin succinate using Pluronic as a binder

Ten parts of solifenacin succinate and 3.4 parts of Pluronic F68 (brand name: Lutrol F68, manufactured by BASF) were dissolved by agitation in a mixed solution of 26.6 parts of methanol and 26.6 parts of water using a stirrer (MGM-66, manufactured by SHIBATA), whereby a drug substance solution was prepared. Then, 60 parts of crystalline cellulose (brand name: Celphere, manufactured by Asahi Chemical Industry Co., Ltd.) were put into a fluidized bed granulator (FLO-1, manufactured by Glatt Co. , Ltd.), and the crystalline cellulose was spray coated with the drug substance solution at an intake air temperature of 54°C, an air flow volume of 0.94 m³/min, a binder solution-spraying rate of 3.0 g/min, and a spraying air pressure of 3.0 kg/cm², whereby a particulate composition of the present invention was obtained.

### Example 10

### A coated product obtained by coating crystalline cellulose with solifenacin succinate and PEG

Ten parts of solifenacin succinate and 1 part of PEG (brand name: Macrogol 6000, manufactured by Sanyo Chemical Industries, Ltd.) were dissolved by agitation in a mixed solution of 16 parts of water and 16 parts of methanol using a stirrer (MGM-66, manufactured by SHIBATA), whereby a drug substance solution was prepared. Then, 60 parts of crystalline cellulose (brand name: Celphere, manufactured by Asahi Chemical Industry Co., Ltd.) were put into a fluidized bed granulator (FLO-1, manufactured by Glatt Co., Ltd.), and the crystalline cellulose was spray coated with the drug substance solution at an intake air temperature of 45°C, an air flow volume of 1.0 m³/min, a binder solution-spraying rate of 2. 0 g/min, and a spraying air pressure of 2.0 kg/cm², whereby a particulate powder was obtained.

### Example 11

### A coated product obtained by coating crystalline cellulose with solifenacin succinate and PEG

Ten parts of solifenacin succinate and 10 parts of PEG (brand name: Macrogol 6000, manufactured by Sanyo Chemical Industries, Ltd.) were dissolved by agitation in a mixed solution of 70 parts of water and 70 parts of methanol using a stirrer (MGM-66, manufactured by SHIBATA), whereby a drug substance solution was prepared. Then, 60 parts of crystalline cellulose (brand name: Celphere, manufactured by Asahi Chemical Industry Co., Ltd.) were put into a fluidized bed granulator (FLO-1, manufactured by Glatt Co., Ltd.), and the crystalline cellulose was spray coated with the drug substance solution at an intake air temperature of 60°C, an air flow volume of 1 m³/min, a binder solution- spraying rate of 6. 5 g/min, and a spraying air pressure of 3.0 kg/cm², whereby a particulate powder was obtained.

### Example 12

### In the case where a different solvent composition was used A coated product obtained by coating crystalline cellulose with solifenacin succinate and PEG

Ten parts of solifenacin succinate and 3.4 parts of PEG (brand name: Macrogol 6000, manufactured by Sanyo Chemical Industries, Ltd.) were dissolved by agitation in 53.2 parts of water using a stirrer (MGM-66, manufactured by SHIBATA), whereby a drug substance solution was prepared. Then, 60 parts of crystalline cellulose (brand name: Celphere, manufactured by Asahi Chemical Industry Co. , Ltd.) were put into a fluidized bed granulator (FLO-1, manufactured by Glatt Co., Ltd.), and the crystalline cellulose was spray coated with the drug substance solution at an intake air temperature of 80°C, an air flow volume of 0. 97 m³/min, a binder solution-spraying rate of 7. 0 g/min, and a spraying air pressure of 3. 0 kg/cm², whereby a particulate powder was obtained.

### Example 13

### A coated product obtained by coating crystalline cellulose with solifenacin succinate and PEG (the content of drug substance: 50%)

Ten parts of solifenacin succinate and 3.4 parts of PEG (brand name: Macrogol 6000, manufactured by Sanyo Chemical Industries, Ltd.) were dissolved by agitation in 26.6 parts of methanol and 26.6 parts of water using a stirrer (MGM-66, manufactured by SHIBATA), whereby a drug substance solution was prepared. Then, 60 parts of crystalline cellulose (brand name: Celphere, manufactured by Asahi Chemical Industry Co., Ltd.) were put into a fluidized bed granulator (FLO-1, manufactured by Glatt Co. , Ltd.) , and the crystalline cellulose was spray coated with the drug substance solution at an intake air temperature of 54°C, an air flow volume of 0.94 m³/min, a binder solution-spraying rate of 3.0 g/min, and a spraying air pressure of 3.0 kg/cm², whereby a particulate powder was obtained. Further, the resulting powder was overcoated with another drug solution prepared at the above-mentioned formulation ratio by using the same device and the same conditions, whereby a particulate powder with a higher content of drug substance (the content of drug substance: 50%) was obtained.

### Comparative example 1

### A coated product obtained by coating a crystalline cellulose core particle with solifenacin succinate using HPMC as a binder

Ten parts of solifenacin succinate and 3.4 parts of hydroxypropylmethyl cellulose 2910 (brand name: TC-5E, manufactured by Shinetsu Chemical Co., Ltd., hereinafter abbreviated as HPMC) were dissolved by agitation in a mixed solution of 26. 6 parts of water and 26.6 parts of methanol using a stirrer (MGM-66, manufactured by SHIBATA), whereby a drug substance solution was prepared. Then, 60 parts of Celphere (manufactured by Asahi Chemical Industry Co., Ltd.) were put into a fluidized bed granulator (FLO-1, manufactured by Glatt Co., Ltd.), and Celphere was spray coated with the drug substance solution at an intake air temperature of 50°C, an air flow volume of 0. 94 m³/min, a binder solution-spraying rate of 7.0 g/min, and a spraying air pressure of 3.0 kg/cm², whereby a particulate composition was obtained.

### Comparative example 2

The particulate composition obtained in Comparative example 1 was subjected to a crystallization treatment by humidification at 25°C and 75% for 12 hours, and then drying at 30°C and 40% for 3 hours, whereby a particulate composition was obtained.

### Comparative example 3

### A coated product obtained by coating a crystalline cellulose core particle with solifenacin succinate using PVP as a binder

Ten parts of solifenacin succinate and 3.4 parts of polyvinylpyrrolidone (brand name: PVP K90, manufactured by BASF, hereinafter abbreviated as PVP) were dissolved by agitation in a mixed solution of 26.6 parts of water and 26.6 parts of methanol using a stirrer (MGM-66, manufactured by SHIBATA), whereby a drug substance solution was prepared. Then, 60 parts of Celphere (manufactured by Asahi Chemical Industry Co., Ltd.) were put into a fluidized bed granulator (FLO-1, manufactured by Glatt Co., Ltd.), and Celphere was spray coated with the drug substance solution at an intake air temperature of 50°C, an air flow volume of 0.97 m³/min, a binder solution-spraying rate of 6 g/min, and a spraying air pressure of 3.0 kg/cm², whereby a particulate composition was obtained.

### Comparative example 4

The particulate composition obtained in Comparative example 3 was subjected to a crystallization treatment by humidification at 25°C and 75% for 12 hours, and then drying at 30°C and 40% for 3 hours, whereby a particulate composition was obtained.

### Comparative example 5

### A coated product obtained by coating a crystalline cellulose core particle with solifenacin succinate using methyl cellulose as a binder

Ten parts of solifenacin succinate and 3.4 parts of methyl cellulose (brand name: Metolose SM100, manufactured by Shinetsu Chemical Co., Ltd., hereinafter abbreviated as MC) were dissolved by agitation in a mixed solution of 53.2 parts of water and 53.2 parts of methanol using a stirrer (MGM-66, manufactured by SHIBATA), whereby a drug substance solution was prepared. Then, 60 parts of Celphere (manufactured by Asahi Chemical Industry Co., Ltd.) were put into a fluidized bed granulator (FLO-1, manufactured by Glatt Co., Ltd.), and Celphere was spray coated with the drug substance solution at an intake air temperature of 55°C, an air flow volume of 0.97 m³/min, a binder solution-spraying rate of 5 g/min, and a spraying air pressure of 3.0 kg/cm², whereby a particulate composition was obtained.

### Comparative example 6

The particulate composition obtained in Comparative example 5 was subjected to a crystallization treatment by humidification at 25°C and 75% for 12 hours, and then drying at 30°C and 40% for 3 hours, whereby a particulate composition was obtained.

### Experimental examples

### <Results of powder X-ray diffraction>

First, the interaction between the drug substance and the polymers was evaluated.

The results of powder X-ray diffraction for spray-dried products comprising PEG and the drug substance used in Example, HPMC or PVP and the drug substance used in Comparative examples (as a device, using RINT 1400: tube bulb: Cu, tube voltage: 40 kV, tube current: 40 mA, scanning rate: 3000°/min (Rigaku. Denki Co.)) are shown in Fig. 1. As the controls, the results for only solifenacin succinate in a crystalline form and PEG are shown together. As a result, any of the products prepared by using PEG shown in Examples exhibits peaks originating in solifenacin succinate in a crystalline form, and it was confirmed that the drug substance in the powder exists as a crystalline form. On the contrary, the samples shown in Comparative examples exhibit a halo pattern typical of an amorphous structure, which revealed that the drug substance exists as an amorphous state.

Further, in Fig. 2, the results of powder X-ray diffraction for the coated product obtained by coating Celphere with PEG shown in Example this time (Example 3) and the coated product obtained by using HPMC shown in Comparative example 1 (as a device, using RINT 2000: tube bulb: Cu, tube voltage: 50 kV, tube current: 300 mA, scanning rate: 60000°/min (Rigaku. Denki Co.)) are shown. As shown in the drawing, even in the coated products, it was confirmed that the sample using PEG exhibits the crystalline peaks originating in solifenacin.

### <Results of preliminary stability test>

The results of a preliminary stability test for these particulate compositions are shown in Tables 1 and 2. The serial measurements of amounts of degradation products after the compositions were stored for a certain period of time were performed by high performance liquid chromatography, and the maximum value among the obtained respective amounts of degradation products is shown (i.e., the production amount of F1, which is a major degradation product). In a test under severe conditions of 40°C and 75% RH, as for the case of using HPMC shown in Comparative example 1, when the humidification treatment was not performed, the degradation product was observed at 0.34% in only a 2-month time course change, which is 8-fold the concentration at the initiation of storage of 0.04%. Further, as for the case of using PVP shown in Comparative example 3, when the humidification treatment was not performed, the degradation product was observed at 0.53% in a 2-month time course change, which exceeds the standard of 0.4%. Further, as for the case of using MC shown in Comparative example 5, when the humidification treatment was not performed, the degradation product was observed at 0.74% in only a 2-month time course change, which is 12-fold the concentration at the initiation of storage of 0.06%. Even in the case where the humidification treatment was performed to promote crystallization, the amount of degradation product for any of Comparative examples 2, 4 and 6 is largeer than that for any of Examples. In the case of using HPMC, the degradation product was observed at 0.92% in only a 2-month time course change, which is 13-fold the concentration at the initiation of storage of 0.07%. Further, in the case of using MC, the degradation product was observed at 11.75% in only a 2-month time course change, which is 11-fold the concentration at the initiation of storage of 1.09%.

On the other hand, as for the samples shown in Examples 1 to 7, whether or not the humidification treatment was performed, the amounts of degradation product at 2 months were 0.2% or lower, and the absolute values were small, between 2 to 390 times less than those of Comparative examples. Further, it was found that solifenacin in any of the pharmaceutical preparations is stable with time because the degree of the change was small. On the other hand, it was found that there is a tendency that the degradation products hardly increase and solifenacin is stable in the case of performing the humidification treatment to promote crystallization as shown in Examples 2, 4, 6 and 8.

The values of Tg (if it does not have a Tg, mp) of the binders used in this time are shown in Table 3, and a linear regression for the relationship between the production amount of a major degradation product (F1 (%)) at 2 months and the Tg (°C) was examined. As a result, as shown in Fig. 3, the squares of the correlation coefficient (R²) were 0.73 in the case where humidification and drying were not performed, and 0. 60 in the case where humidification and drying were performed, which showed a favorable positive correlation.

As described above, it is obvious that the thermodynamic parameters of an additive used in combination are predominant factors for the stability of solifenacin in a pharmaceutical preparation. This phenomenon arises from the fluidity, that is, Tg of a binder as described above, and it was considered that the higher the Tg is, the more easily the amorphous form exists continuously in a pharmaceutical preparation in an amorphous state, therefore the degradation products are easily generated.

The results of the preliminary stability test for particulate compositions containing solifenacin succinate
Storage conditions: 40°C, 75% RH, tightly sealed
Packaging form: HDPE bottle packaging with a metal cap
Testing item: Related substance (production amount of a major degradation product, F1 (%))

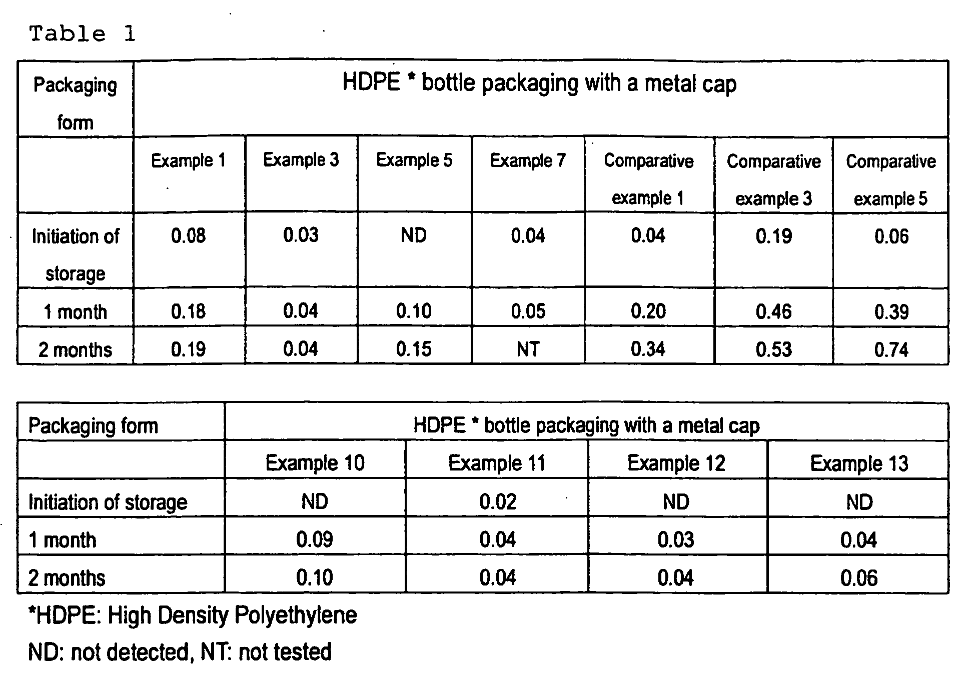

The results of the preliminary stability test for particulate compositions containing solifenacin succinate subjected to a humidification treatment
Storage conditions: 40°C, 75% RH, tightly sealed
Packaging form: HDPE bottle packaging with a metal cap
Testing item: Related substance (production amount of a major degradation product, F1 (%))

**Table 2**

| Packaging form | HDPE * bottle packaging with a metal cap | | | | | | |
|---|---|---|---|---|---|---|---|
| | Example 2 | Example 4 | Example 6 | Example 8 | Comparative example 2 | Comparative example 4 | Comparative example 6 |
| Initiation of storage | 0.09 | 0.03 | 0.03 | 0.02 | 0.07 | 0.23 | 1.09 |
| 1 month | 0.10 | 0.03 | 0.09 | 0.03 | 0.70 | 0.40 | 12.16 |
| 2 months | 0.10 | 0.04 | 0.10 | 0.03 | 0.92 | 0.40 | 11.75 |

The glass transition point Tg (°C) of each binder

**Table 3**

| | Macrogol 6000*, ** | Maltose*, ** | HPC*** | HEC*** | EC*** | PVP*** | HPMC*** | MC*, ** |
|---|---|---|---|---|---|---|---|---|
| Tg (°C) | 60 | 102 | 130 | 137 (135-140) | 157 (152-162) | 174 | 240 | 298 (290-305) |

| | | | | | | | | |
|---|---|---|---|---|---|---|---|---|
| *: Use melting point (mp, °C) as an alternative **: source: "lyakuhin Tenkabutsu Handbook" Yakuji Nippo, Ltd. issued on October 10, 2001 ***: source: "Kobunshi Jiten" Maruzen Co., Ltd., issued on September 20, 1993 | | | | | | | | |

### Industrial Applicability

Technical features of the present invention reside in that a stable particulate pharmaceutical composition can be produced by preparing it using a specific binder in the particulate pharmaceutical composition containing solifenacin or a salt thereof and that it becomes possible to provide a more stable particulate pharmaceutical composition with time by performing a crystallization- promoting treatment such as humidification and drying as needed, which exerts a significant influence on industry. Further, the use of the particulate pharmaceutical composition of the present invention is useful as a technique that can provide various stable pharmaceutical preparations of solifenacin or a salt thereof, for which development as an excellent pharmaceutical product for frequent urination or urinary incontinence has been demanded.

## Claims

1. A stable particulate pharmaceutical composition comprising:
(i) solifenacin or a salt thereof, and
(ii) a binder which stabilizes the solifenacin or salt thereof, the binder having a glass transition point or melting point lower than 174°C;
wherein the binder is one or more of polyethylene glycol, polyethylene oxide, a polyoxyethylene/polyoxypropylene block copolymer, hydroxypropyl cellulose, hydroxyethyl cellulose, ethyl cellulose and maltose.

2. A pharmaceutical composition according to claim 1, wherein the binder is one or more of polyethylene glycol, a polyoxyethylene/polyoxypropylene block copolymer, hydroxypropyl cellulose, hydroxyethyl cellulose and maltose.

3. A pharmaceutical composition according to claim 2, wherein the binder is one or more of polyethylene glycol, a polyoxyethylene/polyoxypropylene block copolymer and hydroxypropyl cellulose.

4. A pharmaceutical composition according to any preceding claim, which is obtainable from a mixture in which the solifenacin or salt thereof and the binder are codissolved and/or suspended.

5. A pharmaceutical composition according to any preceding claim, which has been subjected to a crystallization-promoting treatment selected from a humidification treatment, a microwave irradiation treatment, an ultrasonic irradiation treatment, a low-frequency irradiation treatment and a thermal electron irradiation treatment in order to enhance its stability.

6. A tablet comprising a pharmaceutical composition according to any preceding claim, the tablet being capable of disintegrating in the buccal cavity.

## Patentansprüche

1. Stabile, partikelförmige, pharmazeutische Zusammensetzung, umfassend:
(i) Solifenacin oder ein Salz davon, und
(ii) ein Bindemittel, das Solifenacin oder ein Salz davon stabilisiert, wobei das Bindemittel einen Glasübergangspunkt oder Schmelzpunkt unterhalb von 174°C aufweist;
wobei das Bindemittel eines oder mehrere aus Polyethylenglykol, Polyethylenoxid, einem Polyoxyethylen/Polyoxypropylen Blockcopolymer, Hydroxypropylcellulose, Hydroxyethylcellulose, Ethylcellulose und Maltose ist.

2. Pharmazeutische Zusammensetzung gemäß Anspruch 1, wobei das Bindemittel eines oder mehrere aus Polyethylenglykol, einem Polyoxyethylen/Polyoxypropylen Blockcopolymer, Hydroxypropylcellulose, Hydroxyethylcellulose und Maltose ist.

3. Pharmazeutische Zusammensetzung gemäß Anspruch 2, wobei das Bindemittel eines oder mehrere aus Polyethylenglykol, einem Polyoxyethylen/Polyoxypropylen Blockcopolymer und Hydroxypropylcellulose ist.

4. Pharmazeutische Zusammensetzung gemäß irgendeinem der vorangegangenen Ansprüche, die aus einem Gemisch erhältlich ist, in dem Solifenacin oder ein Salz davon und das Bindemittel zusammen aufgelöst und/oder suspendiert sind.

5. Pharmazeutische Zusammensetzung gemäß irgendeinem der vorangegangenen Ansprüche, die einer kristallisationsfördernden Behandlung, ausgewählt aus einer Befeuchtungsbehandlung, einer Mikrowellenbestrahlung, einer Ultraschallbestrahlung, einer Tieffrequenzbestrahlung und einer thermischen Elektronenbestrahlung unterworfen wurde, um ihre Stabilität zu erhöhen.

6. Tablette, die eine pharmazeutische Zusammensetzung gemäß irgendeinem der vorangegangenen Ansprüche umfasst, wobei die Tablette geeignet ist, sich in der Buccalhöhle aufzulösen.

## Revendications

1. Préparation pharmaceutique granulaire stable comprenant :
(i) de la solifénacine ou un sel de celle-ci, et
(ii) un liant qui stabilise la solifénacine ou un sel de celle-ci, le liant ayant un point de transition vitreuse ou un point de fusion inférieur à 174 °C ;
dans laquelle le liant est un ou plusieurs composés parmi le polyéthylène glycol, l'oxyde de polyéthylène, un copolymère séquence de polyoxyéthylène/polyoxypropylène, l'hydroxypropylcellulose, l'hydroxyéthylcellulose, l'éthylcellulose et le maltose.

2. Préparation pharmaceutique selon la revendication 1, dans laquelle le liant est un ou plusieurs composés parmi le polyéthylène glycol, un copolymère séquence de polyoxyéthylène/polyoxypropylène, l'hydroxypropylcellulose, l'hydroxyéthylcellulose et le maltose.

3. Préparation pharmaceutique selon la revendication 2, dans laquelle le liant est un ou plusieurs composés parmi le polyéthylène glycol, un copolymère séquencé de polyoxyéthylène/polyoxypropylène et l'hydroxypropylcellulose.

4. Préparation pharmaceutique selon l'une quelconque des revendications précédentes, qui peut être obtenue à partir d'un mélange dans lequel la solifénacine ou un sel de celle-ci et le liant sont codissous et/ou en suspension.

5. Préparation pharmaceutique selon l'une quelconque des revendications précédentes, qui a été soumise à un traitement favorisant la cristallisation choisi parmi un traitement d'humidification, un traitement de rayonnement par micro-ondes, un traitement d'irradiation ultrasonique, un traitement d'irradiation à basse fréquence et un traitement d'irradiation thermoélectronique afin d'augmenter sa stabilité.

6. Comprimé comprenant une préparation pharmaceutique selon l'une quelconque des revendications précédentes, le comprimé étant capable de se désintégrer dans la cavité buccale.
